# EUROPEAN PATENT APPLICATION

(11) **EP 4 834 246 A1**
(43) Date of publication of application: **04.11.2026**
(21) Application number: 24911441.4
(22) Date of filing: 27.12.2024
(51) Int. Cl.: A61B 17/122

(54) **LIGATION CLIP ASSEMBLY AND CLIP APPLIER**

(30) Priority: 29.12.2023 CN 202311863270
(71) Applicant: Changzhou Tongchuang Medical Instrument Technology Co., Ltd., Changzhou, Jiangsu 213161 (CN)
(72) Inventor: DING, Weijiang, hangzhou, Jiangsu 213161 (CN); XU, Taofeng, hangzhou, Jiangsu 213161 (CN); CHEN, Maosong, hangzhou, Jiangsu 213161 (CN); MAO, Bo, hangzhou, Jiangsu 213161 (CN)
(74) Representative: Viering, Jentschura & Partner mbB Patent- und Rechtsanwälte
(86) International application number: PCT/CN2024/143097
(87) International publication number: WO 2025/140521

(57) **Abstract**

Provided in the embodiments of the present disclosure are a ligation clip assembly and a clip applier. The ligation clip assembly comprises a plurality of ligation clips. Each of the plurality of ligation clips comprises a first beam, a second beam, and an elastic connecting part connecting the first beam and the second beam. The elastic connecting part is configured to allow the first beam and the second beam to have an open state and a closed state by means of elastic deformation. The plurality of ligation clips comprise a first ligation clip and a second ligation clip which are adjacent to each other, and the first ligation clip and the second ligation clip are connected by means of a connecting member. Therefore, the first ligation clip and the second ligation clip which are adjacent to each other can keep a certain spacing and pulling force during a pushing process, so that the ligation clips are prevented from falling off in a closing process, thereby avoiding the problems such as blockage in the continuous conveying of the ligation clips.

## Description

This application claims priority to Chinese Patent Application No. 202311863270.0 filed on December 29, 2023, the entire disclosure of which is incorporated herein by reference as portion of the present application.

### TECHNICAL FIELD

The present disclosure relates to a ligation clip assembly and a clip applier.

### BACKGROUND

Laparoscopic, endoscopic, and other minimally invasive surgical techniques enable surgeons to perform complex procedures within the body through tiny incisions. Many surgical procedures require ligation of blood vessels during the procedure. For example, many surgical procedures involve cutting blood vessels (such as veins or arteries), which may need to be ligated to reduce bleeding. In some cases, a surgeon may wish to temporarily ligate a blood vessel to reduce blood flow to the surgical site during the procedure. In other cases, a surgeon may wish to permanently ligate a blood vessel.

Vessel ligation may be performed by closing the vessel with a ligation clip or suturing the vessel with sutures. Vessel ligation using sutures requires complex manipulation of needles and suture materials to form the knots required for vessel ligation. Such complex manipulation is time-consuming and difficult to perform, and the difficulty is significantly increased particularly in laparoscopic surgery. Closing a blood vessel with a ligation clip involves a simpler and more reliable procedure.

### SUMMARY

At least one embodiment of the present disclosure provides a ligation clip assembly, the ligation clip assembly comprises a plurality of ligation clips, each of the plurality of ligation clips comprises a first beam, a second beam, and an elastic connecting portion connecting the first beam and the second beam, the elastic connecting portion is configured to allow the first beam and the second beam to have an open state and a closed state through elastic deformation, the plurality of ligation clips comprise a first ligation clip and a second ligation clip that are adjacent to each other, and the first ligation clip and the second ligation clip are connected by a connecting member.

For example, in the ligation clip assembly provided by at least one embodiment of the present disclosure, the first beam comprises a first connecting end away from the elastic connecting portion, the second beam comprises a second connecting end away from the elastic connecting portion, and the first connecting end and the second connecting end are configured to be disconnected or connected to enable the first beam and the second beam to be in the open state or the closed state; and an elastic connecting portion of the first ligation clip is connected to a first connecting end of a first beam of the second ligation clip through the connecting member.

For example, in the ligation clip assembly provided by at least one embodiment of the present disclosure, the first connecting end comprises a hook portion, the second connecting end comprises a protrusion, and the ligation clips are configured such that the protrusion is snapped into an inner side of the hook portion, to achieve connection between the first connecting end and the second connecting end; the connecting member comprises a first end connected to the first ligation clip and a second end connected to the second ligation clip; and the first end is connected to a side of the elastic connecting portion of the first ligation clip away from the first connecting end and the second connecting end, and the second end is connected to an outer side of the hook portion of the second ligation clip.

For example, in the ligation clip assembly provided by at least one embodiment of the present disclosure, the first beam comprises a first boss, and the first boss is provided between the first connecting end and the elastic connecting portion, and provided on a side of the first beam away from the second beam; and the second end is connected to a side of a first boss of the second ligation clip away from an elastic connecting portion of the second ligation clip.

For example, in the ligation clip assembly provided by at least one embodiment of the present disclosure, an axis of the connecting member coincides with a geometric center line of an entirety of the first ligation clip and the second ligation clip when the first beam and the second beam are in a semi-closed state.

For example, in the ligation clip assembly provided by at least one embodiment of the present disclosure, the connecting member is strip-shaped, sheet-shaped, or spherical.

For example, in the ligation clip assembly provided by at least one embodiment of the present disclosure, the connecting member is integrally formed with the first ligation clip and the second ligation clip.

For example, in the ligation clip assembly provided by at least one embodiment of the present disclosure, a contact area between the connecting member and the first ligation clip is different from a contact area between the connecting member and the second ligation clip.

For example, in the ligation clip assembly provided by at least one embodiment of the present disclosure, the connecting member is connected to at least one of the first ligation clip and the second ligation clip in a point-shaped or line-shaped connection.

For example, in the ligation clip assembly provided by at least one embodiment of the present disclosure, the connecting member is spherical, and/or the contact area between the connecting member and the first ligation clip is larger than the contact area between the connecting member and the second ligation clip.

For example, in the ligation clip assembly provided by at least one embodiment of the present disclosure, a through hole penetrating the connecting member and/or the second ligation clip is provided at a connection between the connecting member and the second ligation clip.

For example, in the ligation clip assembly provided by at least one embodiment of the present disclosure, the connecting member is made of a flexible material.

For example, in the ligation clip assembly provided by at least one embodiment of the present disclosure, one of the first ligation clip and the second ligation clip further comprises a pushing portion arranged adjacently and side by side with the connecting member, and the pushing portion is configured to be capable of contacting other one of the first ligation clip and the second ligation clip when subjected to a force to achieve a pushing function.

At least one embodiment of the present disclosure further provides a clip applier, the clip applier comprises a handle assembly, a first tube, the ligation clip assembly provided by the embodiments of the present disclosure, and a pair of jaws, the first tube is connected to the handle assembly; the ligation clip assembly is provided in the first tube; and the jaws are located at a distal end of the first tube.

For example, in the clip applier provided by at least one embodiment of the present disclosure, the ligation clip assembly is in a semi-closed state in the first tube, and an axis of the connecting member of the ligation clip assembly coincides with a geometric center line of the ligation clip assembly.

### BRIEF DESCRIPTION OF DRAWINGS

In order to clearly illustrate the technical solution of the embodiments of the present disclosure, the drawings of the embodiments will be briefly described below. It is obvious that the following described drawings are only related to some embodiments of the present disclosure and are not construed as any limitation to the present disclosure.
Fig. 1 is a schematic perspective structural view of a ligation clip assembly provided by at least one embodiment of the present disclosure;
Fig. 2A is a schematic perspective structural view of a ligation clip in a closed state provided by at least one embodiment of the present disclosure;
Fig. 2B is a schematic side structural view of a ligation clip in a closed state provided by at least one embodiment of the present disclosure;
Fig. 3 is a schematic side view of adjacent ligation clips in a ligation clip assembly connected by a connecting member provided by at least one embodiment of the present disclosure;
Fig. 4 is a schematic side view of a ligation clip assembly in a semi-closed state provided by at least one embodiment of the present disclosure;
Fig. 5 is a schematic top view of a ligation clip assembly in a semi-closed state provided by at least one embodiment of the present disclosure;
Fig. 6 is a schematic perspective structural view of another ligation clip assembly provided by at least one embodiment of the present disclosure;
Fig. 7 is a schematic side view of adjacent ligation clips in another ligation clip assembly connected by a connecting member provided by at least one embodiment of the present disclosure;
Fig. 8 is a schematic top view of adjacent ligation clips in another ligation clip assembly connected by a connecting member provided by at least one embodiment of the present disclosure;
Fig. 9 is a schematic perspective structural view of yet another ligation clip assembly provided by at least one embodiment of the present disclosure;
Fig. 10 is a schematic side view of adjacent ligation clips in yet another ligation clip assembly connected by a connecting member provided by at least one embodiment of the present disclosure;
Fig. 11 is a schematic top view of adjacent ligation clips in yet another ligation clip assembly connected by a connecting member provided by at least one embodiment of the present disclosure; and
Fig. 12 is a schematic structural view of a clip applier provided by at least one embodiment of the present disclosure.

### DETAILED DESCRIPTION

In order to make objectives, technical details, and advantages of the embodiments of the present disclosure more clear, the technical solutions of the embodiments will be described in a clearly and fully understandable way in connection with the drawings related to the embodiments of the present disclosure. Apparently, the described embodiments are just a part but not all of the embodiments of the present disclosure. Based on the described embodiments herein, those skilled in the art can obtain other embodiment(s), without any inventive work, which should be within the scope of the present disclosure.

Unless otherwise defined, technical or scientific terms used in the present disclosure shall have the ordinary meanings as understood by a person having ordinary skill in the art to which the present disclosure belongs. The terms "first", "second", and similar terms used in the present disclosure do not denote any order, quantity, or importance, but are merely used to distinguish different components. Words such as "include" or "comprise" mean that the element or article appearing before the word covers the element or article listed after the word and equivalents thereof, without excluding other elements or articles. Words such as "connected" or "coupled" are not limited to physical or mechanical connections, but may include electrical connections, whether direct or indirect. "Upper", "lower", "left", "right", and the like are used merely to indicate relative positional relationships, and when the absolute position of the object being described is changed, the relative positional relationships may change accordingly. The term "proximal end" refers to a portion of the involved component or structure that is closer to the clinician, and the term "distal end" refers to a portion of the involved component or structure that is farther from the clinician. The term " multiple/plurality" means two or more.

During the process of closing a blood vessel with a ligation clip, a clip applier (or ligation forceps) is typically used to carry the ligation clip and insert it together through a trocar into a designated position in the body cavity. In use, the ligation clip can be positioned on the blood vessel to be ligated, and the clip applier is controlled outside the body to close the ligation clip.

However, in the conventional operation mode, only one ligation clip can be picked up and clamped at a time. If multiple ligation clips are needed, the clip applier needs to be removed from the body cavity, a new ligation clip is picked up outside the body cavity, and then the clip applier is reinserted into the body cavity to clamp the ligation clip. Clinically, each surgical procedure often requires several ligation clips, and repeated picking up of ligation clips greatly reduces surgical efficiency and increases surgical risks.

In some embodiments, a method of continuous firing of ligation clips can be used, that is, multiple ligation clips are mounted on one clip applier, so that the operator does not need to withdraw the clip applier from the body cavity and can continuously clamp multiple ligation clips.

However, in current methods of continuous firing of ligation clips, there are often many problems. Multiple single ligation clips are relatively independent without structural restrictions on the positional relationship between the ligation clips, and due to the need to transport multiple consecutive single ligation clips, the transport structure is relatively complex to ensure the distance relationship and reliable positioning of the multiple single ligation clips, so failures are prone to occur during use. For example, when a ligation clip is not properly provided in the jaws, the ligation clip may fall off the jaws or fail to close; the track for transporting the ligation clip may be blocked, resulting in failure of ligation clip closure; the structure for transporting the ligation clip is precise and complex, which is likely to cause damage to the ligation clip body during transport, thereby affecting the close performance; and deformation of the material of the ligation clip itself may easily lead to ineffective fixation of the ligation clip in the jaws of the clip applier.

In summary, there are several defects in the continuous firing of ligation clips, and an optimized structure is needed to safely and reliably transport and close ligation clips in sequence, and minimize damage to blood vessels.

At least one embodiment of the present disclosure provides a ligation clip assembly and a clip applier. The ligation clip assembly includes a plurality of ligation clips, each of the plurality of ligation clips includes a first beam, a second beam, and an elastic connecting portion connecting the first beam and the second beam, the elastic connecting portion is configured to allow the first beam and the second beam to have an open state and a closed state through elastic deformation, the plurality of ligation clips include a first ligation clip and a second ligation clip that are adjacent to each other, and the first ligation clip and the second ligation clip are connected by a connecting member.

Thus, in the above ligation clip assembly provided by the embodiments of the present disclosure, adjacent first and second ligation clips can maintain a certain spacing and pulling force during pushing, thereby preventing the ligation clips from falling off during closure, preventing blockage of the ligation clips during continuous transport, and simplifying the structure of the clip applier, thereby avoiding damage to the ligation clips and the like.

The ligation clip assembly and clip applier provided by the embodiments of the present disclosure are described below through several specific embodiments.

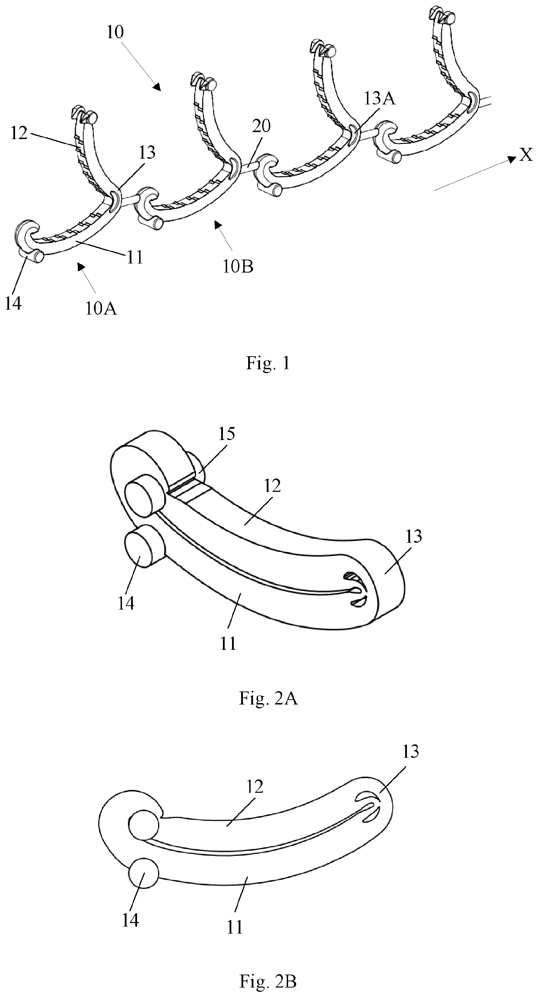

At least one embodiment of the present disclosure provides a ligation clip assembly. Fig. 1 shows a schematic perspective structural view of the ligation clip assembly. As shown in Fig. 1, the ligation clip assembly includes a plurality of ligation clips 10, each ligation clip 10 includes a first beam 11, a second beam 12, and an elastic connecting portion 13 connecting the first beam 11 and the second beam 12, and the elastic connecting portion 13 is configured to allow the first beam 11 and the second beam 12 to have an open state and a closed state through elastic deformation.

For example, Fig. 1 shows a schematic perspective structural view of a plurality of ligation clips 10 in an open state, and Fig. 2A and Fig. 2B respectively show a schematic perspective view and a schematic side view of a single ligation clip 10 of the ligation clip assembly in the closed state. For example, as shown in Fig. 1, each ligation clip 10 has a hollow portion 13A at the elastic connecting portion 13, and the hollow portion 13A is, for example, in the form of a crescent or a strip shape to facilitate corresponding elastic deformation of the elastic connecting portion 13 when realizing the open state and the closed state.

For example, when the elastic connecting portion 13 undergoes elastic deformation to realize the closed state, the inner side of the hollow portion 13A (i.e., the side close to the closed position, the left side in Fig. 2B) is compressed, and the outer side of the hollow portion 13A (i.e., the side away from the closed position, the right side in Fig. 2B) is stretched; and when the elastic connecting portion 13 undergoes elastic deformation to realize the open state, the inner and outer sides of the hollow portion 13A are both in a relaxed state, i.e., an unstressed state.

For example, as shown in Fig. 1, the plurality of ligation clips 10 include a first ligation clip 10A and a second ligation clip 10B that are adjacent to each other, and the first ligation clip 10A and the second ligation clip 10B are connected by a connecting member 20. For example, the plurality of ligation clips 10 included in the ligation clip assembly are arranged in sequence along a first direction X, every two adjacent ligation clips 10 are connected by a connecting member 20, and the connection manner of every two adjacent ligation clips 10 is substantially the same.

Thus, the plurality of ligation clips 10 included in the ligation clip assembly are connected end to end in sequence through the connecting members 20 to form an integral body. When the ligation clip assembly is provided in the clip applier, adjacent ligation clips can maintain a certain spacing and pulling force during pushing in the clip applier, thereby preventing the ligation clips from falling off during closure, preventing blockage of the ligation clips during continuous transport, and simplifying the structure of the clip applier, thereby avoiding damage to the ligation clips caused by the complex structure; for example, when a previous ligation clip is used, the connection between it and a subsequent ligation clip can be broken, thereby realizing independent use of each ligation clip.

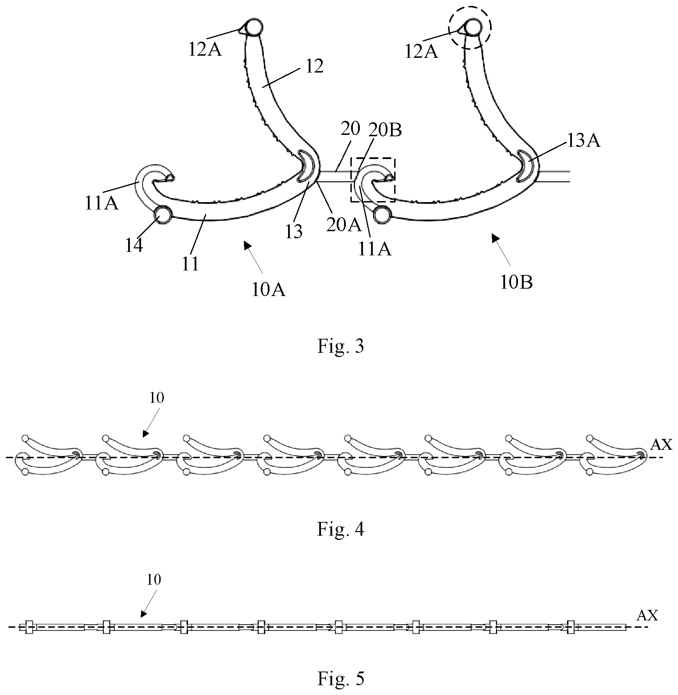

For example, Fig. 3 shows a schematic side view of adjacent first ligation clip 10A and second ligation clip 10B. As shown in Fig. 3, the first beam 11 includes a first connecting end 11A away from the elastic connecting portion 13, the second beam 12 includes a second connecting end 12A away from the elastic connecting portion 13, and the first connecting end 11A and the second connecting end 12A are configured to be connected or disconnected to realize the open state and the closed state. For example, the elastic connecting portion 13 of the first ligation clip 10A is connected to the first connecting end 11A of the first beam 11 of the second ligation clip 10B through the connecting member 20.

For example, in some examples, as shown in Fig. 3, the first connecting end 11A includes a hook portion, as shown by the dashed box; and the second connecting end 12A includes a protrusion, as shown by the dashed circle, and the protrusion is configured to be snapped into the inner side of the hook portion (i.e., the side close to the elastic connecting portion 13) to realize the connection between the first connecting end 11A and the second connecting end 12A.

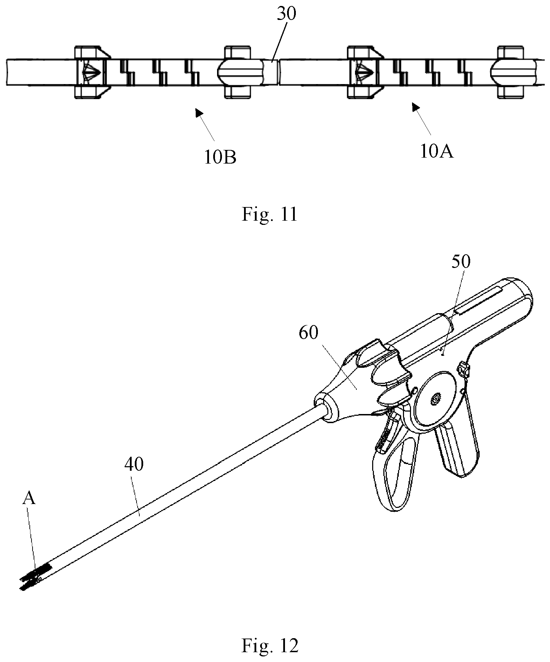

For example, in some examples, as shown in Fig. 2A, the second connecting end 12A may further include bosses 15 provided on opposite sides of the protrusion, for example, in the direction perpendicular to the first direction X in Fig. 1, and the bosses 15 are shown as cylinders in the figure to have smooth surfaces. When the protrusion is snapped into the inner side of the hook portion, the bosses 15 can limit the snap position and prevent the protrusion from disengaging from the hook portion. For example, when the ligation clip assembly is provided in the clip applier, the bosses 15 may also be used for positioning in the clip applier, and when the ligation clip is pushed to the jaws A (see Fig. 12) of the clip applier, the bosses 15 may also be used for positioning in the jaws A of the clip applier.

For example, as shown in Fig. 3, the connecting member 20 includes a first end 20A connected to the first ligation clip 10A and a second end 20B connected to the second ligation clip 10B. The first end 20A is connected to a side of the elastic connecting portion 13 of the first ligation clip 10A away from the first connecting end 11A and the second connecting end 12A of the first ligation clip 10A (i.e., the outer side of the elastic connecting portion 13 of the first ligation clip 10A), and the second end 20B is connected to the outer side of the hook portion of the second ligation clip 10B (i.e., the side away from the elastic connecting portion 13 of the second ligation clip 10B).

For example, as shown in Fig. 1 and Fig. 3, the first beam 11 includes a first boss 14 provided between the first connecting end 11A and the elastic connecting portion 13 and provided on a side of the first beam 11 away from the second beam 12. For example, when the ligation clip assembly is provided in the clip applier, the first boss 14 can be used for positioning in the clip applier, and when the ligation clip is pushed to the jaws of the clip applier, the first boss 14 can also be used for positioning in the jaws of the clip applier.

For example, in some examples, the first boss 14 may be a cylindrical structure, so as to have a smooth surface.

Thus, the positional stability of the ligation clip in the pushing device and the jaws of the clip applier can be realized through the protrusion of the second beam 12 and the first boss 14 of the first beam 11.

For example, as shown in Fig. 3, the second end 20B of the connecting member 20 is connected to a side of the first boss 14 of the second ligation clip 10B away from the elastic connecting portion 13 of the second ligation clip 10B. Through the above arrangement of the connecting member 20, the position of the connecting member 20 is biased toward the geometric center line of the plurality of connected ligation clips, making the pushing process of the plurality of ligation clips more stable.

For example, when the ligation clip assembly is provided in the clip applier, the ligation clip 10 is in a semi-closed state. Fig. 4 shows a schematic side view of the ligation clip 10 in the ligation clip assembly in the semi-closed state, and Fig. 5 shows a schematic top view of the ligation clip 10 in the ligation clip assembly in the semi-closed state. As shown in Fig. 4 and Fig. 5, when the first beam 11 and the second beam 12 are in the semi-closed state, the axis of the connecting member 20 coincides with the geometric center line of the entirety of the first ligation clip 10A and the second ligation clip 10B, which is line AX in the figures. The semi-closed state refers to a state where the first beam 11 and the second beam 12 are not completely closed, i.e., the first connecting end 11A and the second connecting end 12A are not connected, but the second beam 12 approaches the first beam 11 due to an external force.

For example, when the ligation clip assembly is provided in the clip applier, the ligation clip is compressed to the semi-closed state due to the diameter limitation of the tube of the clip applier. As shown in Fig. 4, the second beam 12 of each ligation clip 10 is pressed, so that the included angle between the first beam 11 and the second beam 12 of each ligation clip 10 is reduced to form the semi-closed state as shown in Fig. 4, and each ligation clip 10 can be pushed in this state. In this case, the axis of the connecting member 20 coincides with the geometric center line of the entirety of the first ligation clip 10A and the second ligation clip 10B, so that the pushing force of the ligation clip assembly is balanced without causing a rotation tendency of a certain ligation clip 10, thereby facilitating the structural stability of the ligation clip assembly during pushing.

For example, in some embodiments, each ligation clip 10 is an integral structure, for example, formed as an integral structure by an injection molding process or the like. For example, in some embodiments, the connecting member 20 is also integrally formed with the first ligation clip 10A and the second ligation clip 10B, for example, integrally formed by an injection molding process or the like, so that the ligation clip assembly is formed as an integral structure, and there is no obvious connection interface between the connecting member 20 and the ligation clip 10 connected thereto. Thus, the ligation clip assembly is easier to form, and the formed structure is more accurate and stable, and the connecting member 20 is easier to be broken when it is necessary to disconnect adjacent ligation clips during use.

For example, in other embodiments, the connecting member 20 and the ligation clip 10 connected thereto may also be connected by other forms such as bonding or clamping. However, compared with the above integral molding, the connection strength by connection forms such as bonding or clamping needs to be reasonably designed to ensure the connection effect and also meet the requirement of easy breaking during use.

For example, in some embodiments, the connecting member 20 is in the shape of a strip (e.g., a column, etc.), a sheet, a sphere (including a hemisphere with an arc surface, etc.), or the like.

For example, in the embodiments of Fig. 1 and Fig. 2A, the connecting member 20 is in the shape of a strip, e.g., a cylinder. The first end 20A of the connecting member 20 is connected to a position of the elastic connecting portion 13 of the first ligation clip 10A biased toward the first beam 11, which is shown as a position slightly below the center of the elastic connecting portion 13 in Fig. 3, and the second end 20B of the connecting member 20 is connected to the outer side of the hook portion of the second ligation clip 10B.

For example, in some embodiments, the connection strength between the connecting member 20 and the first ligation clip 10A is different from the connection strength between the connecting member 20 and the second ligation clip 10B, so that the side with weaker connection strength is easier to be broken, thereby disconnecting the first ligation clip 10A from the second ligation clip 10B in a simple manner during use.

For example, in some embodiments, the contact area between the connecting member 20 and the first ligation clip 10A is different from the contact area between the connecting member 20 and the second ligation clip 10B. For example, in some examples, the diameter of the connecting member 20 shows a gradual change (e.g., gradually increasing or decreasing) between the first ligation clip 10A and the second ligation clip 10B, so that it is easy to be broken at the side with a smaller diameter.

In the embodiments of the present disclosure, the contact area between the connecting member 20 and the ligation clip may be an area of a cross-section formed by disconnecting the connecting member 20 from the ligation clip at a contact/connection position between the connecting member 20 and the ligation clip.

For example, in some embodiments, the contact area between the connecting member 20 and the first ligation clip 10A is 0.38 mm² to 1.2 mm², such as 0.5 mm², 0.7 mm², 0.9 mm², or 1.1 mm², etc., and the contact area between the connecting member 20 and the second ligation clip 10B is less than or equal to 0.12 mm², such as 0.05 mm², 0.08 mm², 0.10 mm², or 0.12 mm², etc. Thus, the contact area between the connecting member 20 and the second ligation clip 10B is smaller, and the connection between the connecting member 20 and the second ligation clip 10B are easier to be broken; and the connection between the connecting member 20 and the first ligation clip 10A is more stable, which is conducive to providing connection strength and pushing force.

For example, in other embodiments, the connection strength between the connecting member 20 and the first ligation clip 10A may also be approximately the same as the connection strength between the connecting member 20 and the second ligation clip 10B. In this case, the contact areas between the connecting member 20 and the first ligation clip 10A and between the connecting member 20 and the second ligation clip 10B are both 0.38 mm² to 1.2 mm² to form a larger and more stable pushing force between adjacent ligation clips, and the connection can also be disconnected by an auxiliary tool after use; alternatively, the contact areas between the connecting member 20 and the first ligation clip 10A and between the connecting member 20 and the second ligation clip 10B are both less than or equal to 0.12 mm². In this case, a certain pushing force is still maintained between adjacent ligation clips, and adjacent ligation clips can be disconnected by slight pulling or twisting after use.

For example, according to different clip applier structures, when the contact areas between the connecting member 20 and the first ligation clip 10A and between the connecting member 20 and the second ligation clip 10B are both less than or equal to 0.12 mm², the connecting member 20 may form a point-shaped (the connecting member 20 has a very small diameter) or strip-shaped/line-shaped (the connecting member 20 has a very thin thickness) connection with the first ligation clip 10A and the second ligation clip 10B, so that adjacent ligation clips can be disconnected by slight pulling or twisting.

For example, in still other embodiments, a portion of the connecting member 20 between the first ligation clip 10A and the second ligation clip 10B may have a smaller diameter for easy breaking; in this case, the contact areas between the connecting member 20 and the first ligation clip 10A and between the connecting member 20 and the second ligation clip 10B may be the same or different.

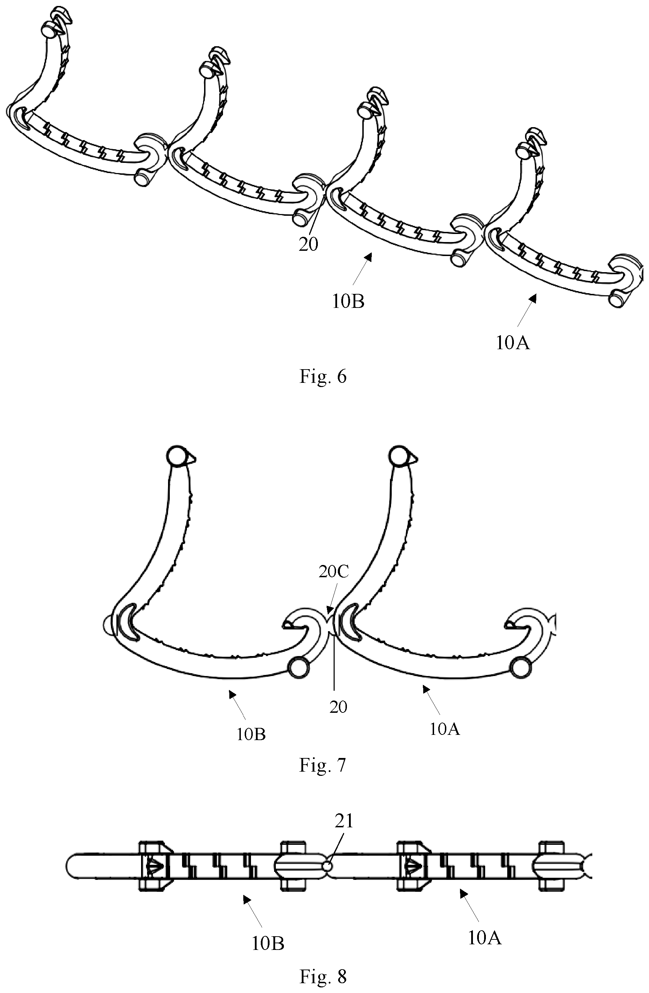

For example, Fig. 6 shows a schematic perspective structural view of another ligation clip assembly provided by at least one embodiment of the present disclosure, Fig. 7 shows a schematic side view of the ligation clip assembly, and Fig. 8 shows a schematic top view of the ligation clip assembly. As shown in Fig. 6-Fig. 8, in the present embodiment, the connecting member 20 is spherical (including a sphere-like shape, an incomplete spherical shape such as a hemisphere, 3/4 sphere, etc.), e.g., hemispherical. The contact area between the connecting member 20 and the first ligation clip 10A is larger than the contact area between the connecting member 20 and the second ligation clip 10B, so that the connection strength between the connecting member 20 and the first ligation clip 10A is greater than the connection strength between the connecting member 20 and the second ligation clip 10B, thereby facilitating breaking at the connection position between the connecting member 20 and the second ligation clip 10B.

For example, as shown in Fig. 7, when the connecting member 20 is spherical, a V-shaped structure is formed at the connection position 20C between the connecting member 20 and the second ligation clip 10B (i.e., in the cross-section of Fig. 7, the surfaces of the connecting member 20 and the second ligation clip 10B at the connection position 20C have a V-shaped cross-sectional structure). In this case, when disconnecting the connecting member 20 from the second ligation clip 10B, on the one hand, the connection strength between the connecting member 20 and the second ligation clip 10B is lower and easier to be broken, i.e., the first ligation clip 10A is separated from the second ligation clip 10B at the position with the weakest connection strength to reduce the separation difficulty; on the other hand, after the connecting member 20 and the second ligation clip 10B are broken at the bottom of the V-shaped structure, the cross-sections of the connecting member 20 and the second ligation clip 10B form obtuse angles without generating an acute angle (sharp corner) structure, so as to avoid damage to tissues.

For example, in some embodiments, a through hole 21 penetrating the connecting member 20 and/or the second ligation clip 10B may be further provided at the connection position 20C between the connecting member 20 and the second ligation clip 10B. This can further weaken the connection strength between the connecting member 20 and the second ligation clip 10B.

For example, the through hole 21 may be formed in the connecting member 20, or in the second ligation clip 10B, or partially in the connecting member 20 and partially in the second ligation clip 10B. The cross-section of the through hole 21 may be circular, rhombic, triangular, or the like, and the embodiments of the present disclosure not specifically limit this.

For example, in some examples, as shown in Fig. 8, the through hole 21 is partially formed in the connecting member 20 and partially in the second ligation clip 10B, and the diameter or diagonal of the through hole 21 is located at the connection position between the connecting member 20 and the second ligation clip 10B, so that the connection strength at the connection position between the connecting member 20 and the second ligation clip 10B is weaker.

For example, in the embodiments of the present disclosure, the spherical shape of the connecting member 20 includes cases where the connecting member 20 has an arc surface, a hemispherical shape, and other modified forms, as long as the connecting member 20 is connected to the ligation clip via an arc surface.

Thus, in the above embodiments, the connecting member 20 is formed in an arc-surface bridging manner, and the arc-surface bridging makes the connection structure more compact, for example, having a smaller length (the transverse dimension in Fig. 7) between adjacent ligation clips 10. When the arc surface of the connecting member 20 is connected to the rear second ligation clip 10B, a combination of two spherical surfaces is formed, and a "V"-shaped guide groove is naturally formed at the combination position. When cutting to disconnect two adjacent ligation clips, the "V"-shaped guide groove can help locate the cutting position. After cutting, because both sides of the cross-section are spherical surfaces with larger diameters, the cross-section will not produce acute angles, reducing the risk of the cross-section scratching human tissues. In addition, a through hole 21 is formed at the bridging position between the arc surface of the connecting member 20 and the rear second ligation clip 10B, making the cutting action smoother and the cross-section smoother.

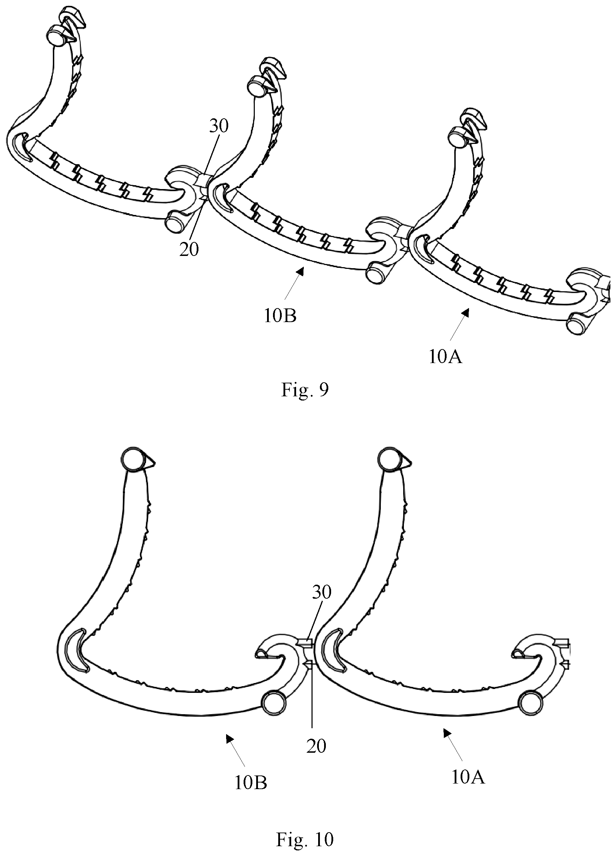

For example, Fig. 9 shows a schematic perspective structural view of yet another ligation clip assembly provided by at least one embodiment of the present disclosure, Fig. 10 shows a schematic side view of the ligation clip assembly, and Fig. 11 shows a schematic top view of the ligation clip assembly.

As shown in Fig. 9-Fig. 11, in the present embodiment, the connecting member 20 is made of a flexible material. In another embodiment, the connecting member 20 may also be a rigid member or a composite member combining soft and hard materials. The connecting member 20 is connected to at least one of the first ligation clip 10A and the second ligation clip 10B in a point-shaped or line-shaped connection.

In the present embodiment, the connecting member 20 is connected to one of the first ligation clip 10A and the second ligation clip 10B in a point-shaped or line-shaped connection, and connected to the other in a surface-shaped connection, i.e., the connection area between the connecting member 20 and one of the first ligation clip 10A and the second ligation clip 10B is smaller, so that the connection strength is weaker. For example, as shown in Fig. 10, the connecting member 20 is connected to the first ligation clip 10A in a point-shaped or line-shaped connection, i.e., the cross-section of the connecting member 20 at the connection position with the first ligation clip 10A is point-shaped or line-shaped, for example, the cross-section area is less than or equal to 0.12 mm², such as 0.05 mm², 0.08 mm², 0.10 mm², or 0.12 mm², etc. The connecting member 20 is connected to the second ligation clip 10B in a surface-shaped connection, i.e., the cross-section of the connecting member 20 at the connection position with the second ligation clip 10B has a certain area, for example, the cross-section area ranges from 0.38 mm² to 1.2 mm², such as 0.5 mm², 0.7 mm², 0.9 mm², or 1.1 mm², etc. Thus, the connection strength between the connecting member 20 and the first ligation clip 10A is weaker than the connection strength between the connecting member 20 and the second ligation clip 10B. Thus, during use, the connection can be broken at the connection between the connecting member 20 and the first ligation clip 10A.

For example, to maintain the spacing and pushing force between the first ligation clip 10A and the second ligation clip 10B, one of the first ligation clip 10A and the second ligation clip 10B (shown as the second ligation clip 10B in the figure) further includes a pushing portion 30 provided adjacent to and side by side with the connecting member 20, and the pushing portion 30 is configured to be able to contact the other of the first ligation clip 10A and the second ligation clip 10B (shown as the first ligation clip 10A in the figure) when subjected to a force to achieve a pushing function.

For example, the pushing portion 30 is provided on a ligation clip located at the rear during pushing. For example, in the embodiment of Fig. 10, the ligation clip assembly is pushed to move distally, i.e., toward the opening direction of the ligation clips, and in this case, the pushing portion 30 is provided on the second ligation clip 10B located at the rear.

For example, in the above embodiments, the pushing portion 30 may be an integral structure with the first ligation clip 10A and the second ligation clip 10B, for example, integrally formed in a process such as injection molding, and formed of a rigid material, such as polyoxymethylene. For example, the flexible material used for the connecting member 20 may be a soft material such as silicone rubber, thermoplastic elastomer (TPE), or cotton thread, and its hardness is less than the hardness of the ligation clip, and the embodiments of the present disclosure do not specifically limit this.

For example, during the pushing process of the pushing portion 30, the contact area between the pushing portion 30 and the first ligation clip 10A may be 0.38 mm² to 1.2 mm², such as 0.5 mm², 0.7 mm², 0.9 mm², or 1.1 mm², etc., which can effectively prevent the ligation clip from deflecting during pushing and facilitate the stability of the pushing process.

Thus, in the above embodiments, the connection between adjacent first ligation clip 10A and second ligation clip 10B is formed as a composite structure combining soft and hard materials. The hard material (i.e., the pushing portion 30) may still use a polymer material the same as or similar in hardness to the ligation clip body, and the soft material (i.e., the connecting member 20) may be selected from a flexible material with hardness less than the hardness of the ligation clip body. The hard material is provided at the front end of the ligation clip but is not directly connected to the tail of the front ligation clip. When the ligation clips are pushed forward, the hard material contacts the tail of the front ligation clip forward and pushes it forward. During the pushing process, the larger contact area between the hard material and the tail of the front ligation clip can effectively prevent the ligation clip from deflecting during the pushing process; the soft material connects the front and rear ligation clips, enabling the ligation clips to be transported in a bent state and preventing accidental disengagement of the front and rear ligation clips; and when the front and rear ligation clips need to be cut, the soft material can be easily cut, and due to the properties of the soft material, even if an acute angle is generated on the cross-section, there is no risk of scratching human tissues.

In summary, in the embodiments of the present disclosure, the ligation clip assembly is formed as connected ligation clips, and the connected ligation clips include a plurality of ligation clips connected in sequence. Pushing only the first distal ligation clip enables the plurality of ligation clips to be pushed together. Compared with the conventional case where a single ligation clip is pushed independently and repositioned each time, the combined pushing of the ligation clip assembly can avoid problems such as difficulty in controlling the positional relationship between the ligation clips and blockage of the ligation clips during continuous transport.

In addition, the connecting member of the ligation clip assembly may still select the same material as the ligation clip, has good elasticity, and can be molded with the ligation clip in one step, enabling the bending movement of the ligation clip assembly in the clip applier without additional auxiliary structures. Furthermore, during the pushing and closing process of the ligation clip assembly, the tail end of the previous ligation clip is always connected and restricted by the rear ligation clip, thereby preventing the ligation clip located at the jaws of the clip applier from falling off the jaws and causing surgical safety risks. On the other hand, the ligation clip assembly can realize the overall transportation of the ligation clips by pushing the end or individual positions, without the need to adapt a precise pushing structure for each ligation clip. Such a simple structure is not prone to failure, can ensure smooth and rapid use of the ligation clips, and can avoid damage to the ligation clips caused by jamming during the pushing process, thereby greatly reducing the possibility of ligation clip failure during surgery.

In addition, compared with a single ligation clip, connected ligation clips are simple to assemble (e.g., assembled in a clip applier, etc.) without the need to adjust the assembly state of each ligation clip one by one; and the ligation clip assembly can be integrally processed and molded, with simple process and low cost.

At least one embodiment of the present disclosure further provides a clip applier, which includes a pushing device and the ligation clip assembly of the embodiments of the present disclosure.

For example, Fig. 12 shows a schematic structural view of a clip applier provided by at least one embodiment of the present disclosure. As shown in Fig. 12, the clip applier includes a first tube 40 and the ligation clip assembly provided by the embodiments of the present disclosure, and the ligation clip assembly is located in the first tube 40 and can be pushed in the first tube 40.

For example, as shown in Fig. 12, the clip applier further includes a pair of jaws A, a handle assembly 50, and a rotating assembly 60. The proximal end of the first tube 40 is connected to the handle assembly 50. The jaws A are provided at the distal end, such as an operating end, of the first tube 40, and each ligation clip in the ligation clip assembly can be pushed to the jaws A in sequence in the first tube 40 and closed and used.

For example, the rotating assembly 60 is connected to the first tube 40 and can control the rotation of the first tube 40 and the jaws A, thereby controlling the angle of the jaws A relative to a target object; and the handle assembly 50 can be held by hand, and may further include other operating components, and the embodiments of the present disclosure do not specifically limit this.

For example, the ligation clip assembly is in the semi-closed state in the first tube 40, and the axis of the connecting member 20 of the ligation clip assembly in the semi-closed state coincides with the geometric center line of the ligation clip assembly.

For example, when the ligation clip assembly is provided in the first tube 40, referring to Fig. 4, each ligation clip included in the ligation clip assembly is in the semi-closed state, and the axis of the connecting member 20 coincides with the geometric center line of the entirety of the ligation clip assembly, which is line AX in the figure. This can balance the pushing force of the ligation clip assembly without causing a rotation tendency of a certain ligation clip 10, and is conducive to the structural stability of the ligation clip assembly during the pushing process.

The following statements should be noted:
The accompanying drawings involve only the structure(s) in connection with the embodiment(s) of the present disclosure, and other structure(s) can be referred to common design(s).

For the sake of clarity, in the drawings used to describe the embodiments disclosed herein, the thicknesses of the layers or regions are enlarged or scaled down, that is, these drawings are not drawn to actual scale.

In case of no conflict, features in one embodiment or in different embodiments can be combined to obtain new embodiments.

What have been described above are only specific implementations of the present disclosure, the protection scope of the present disclosure is not limited thereto, and the protection scope of the present disclosure should be based on the protection scope of the claims.

## Claims

1. A ligation clip assembly, comprising a plurality of ligation clips,
wherein each of the plurality of ligation clips comprises a first beam, a second beam, and an elastic connecting portion connecting the first beam and the second beam, the elastic connecting portion is configured to allow the first beam and the second beam to have an open state and a closed state through elastic deformation,
the plurality of ligation clips comprise a first ligation clip and a second ligation clip that are adjacent to each other, and the first ligation clip and the second ligation clip are connected by a connecting member.

2. The ligation clip assembly according to claim 1, wherein the first beam comprises a first connecting end away from the elastic connecting portion, the second beam comprises a second connecting end away from the elastic connecting portion, and the first connecting end and the second connecting end are configured to be disconnected or connected to enable the first beam and the second beam to be in the open state or the closed state; and
an elastic connecting portion of the first ligation clip is connected to a first connecting end of a first beam of the second ligation clip through the connecting member.

3. The ligation clip assembly according to claim 2, wherein the first connecting end comprises a hook portion, the second connecting end comprises a protrusion, and the ligation clips are configured such that the protrusion is snapped into an inner side of the hook portion, to achieve connection between the first connecting end and the second connecting end;
the connecting member comprises a first end connected to the first ligation clip and a second end connected to the second ligation clip; and
the first end is connected to a side of the elastic connecting portion of the first ligation clip away from the first connecting end and the second connecting end, and the second end is connected to an outer side of the hook portion of the second ligation clip.

4. The ligation clip assembly according to claim 3, wherein the first beam comprises a first boss, and the first boss is provided between the first connecting end and the elastic connecting portion, and provided on a side of the first beam away from the second beam; and
the second end is connected to a side of a first boss of the second ligation clip away from an elastic connecting portion of the second ligation clip.

5. The ligation clip assembly according to any one of claims 1 to 4, wherein an axis of the connecting member coincides with a geometric center line of an entirety of the first ligation clip and the second ligation clip when the first beam and the second beam are in a semi-closed state.

6. The ligation clip assembly according to any one of claims 1 to 5, wherein the connecting member is strip-shaped, sheet-shaped, or spherical.

7. The ligation clip assembly according to any one of claims 1 to 6, wherein the connecting member is integrally formed with the first ligation clip and the second ligation clip.

8. The ligation clip assembly according to any one of claims 1 to 7, wherein a contact area between the connecting member and the first ligation clip is different from a contact area between the connecting member and the second ligation clip.

9. The ligation clip assembly according to claim 8, wherein the connecting member is connected to at least one of the first ligation clip and the second ligation clip in a point-shaped or line-shaped connection.

10. The ligation clip assembly according to claim 8, wherein the connecting member is spherical, and/or
the contact area between the connecting member and the first ligation clip is larger than the contact area between the connecting member and the second ligation clip.

11. The ligation clip assembly according to claim 10, wherein a through hole penetrating the connecting member and/or the second ligation clip is provided at a connection between the connecting member and the second ligation clip.

12. The ligation clip assembly according to any one of claims 8 to 11, wherein the connecting member is made of a flexible material.

13. The ligation clip assembly according to claim 12, wherein one of the first ligation clip and the second ligation clip further comprises a pushing portion arranged adjacently and side by side with the connecting member, and the pushing portion is configured to be capable of contacting other one of the first ligation clip and the second ligation clip when subjected to a force to achieve a pushing function.

14. A clip applier, comprising:
a handle assembly;
a first tube connected to the handle assembly;
the ligation clip assembly according to any one of claims 1 to 13, provided in the first tube; and
a pair of jaws located at a distal end of the first tube.

15. The clip applier according to claim 14,
wherein the ligation clip assembly is in a semi-closed state in the first tube, and
an axis of the connecting member of the ligation clip assembly coincides with a geometric center line of the ligation clip assembly.
